# EUROPEAN PATENT APPLICATION

(11) **EP 1 803 450 A1**
(43) Date of publication of application: **04.07.2007**
(21) Application number: 06100029.5
(22) Date of filing: 03.01.2006
(51) Int. Cl.: A61K 9/50, A61K 31/4439, A61K 31/43, A61K 31/7048, A61P 1/04, A61P 31/04

(54) **Pharmaceutical compositions for the eradication of helicobacter pylori**

(71) Applicant: FERRER INTERNACIONAL, S.A., 08028 Barcelona (ES)
(72) Inventor: Miralles, Ricardo, 08190 Sant Cugat del Vallès (ES); Torres, Jesús, 08022 Barcelona (ES); Suñé, Josep M., 08028 Barcelona (ES)
(74) Representative: Barlocci, Anna

(57) **Abstract**

A pellet composition for oral administration comprising simultaneously a proton pump inhibitor, a clarithromycin compound and an amoxicillin compound. Said compositions are suitable to be filled in sachets and are useful in the treatment of disorders associated with *Helicobacter* bacteria.

## Description

### Technical field

The present invention relates to the field of pharmaceutical technology and describes a novel administration form comprising simultaneously a proton pump inhibitor (H+/K+ ATPase inhibitor), a clarithromycin compound and an amoxicillin compound suitable for the treatment of gastrointestinal infections caused or mediated by *Helicobacter pylori.* More specifically the administration form comprises pellets filled into sachets for oral administration.

### Background of the invention

The majority of peptic ulcers are caused by the *Helicobacter pylori* bacterium. A combination of antibiotics and other drugs is the most effective treatment for *Helicobacter pylori*-mediated peptic ulcers.

*Helicobacter pylori* weakens the protective mucous coating of the stomach and duodenum, which allows acid to get through to the sensitive lining beneath. Both the acid and the bacteria irritate the lining and cause an ulcer.
*Helicobacter pylori* is able to survive in stomach acid because it secretes enzymes that neutralize the acid. This mechanism allows *Helicobacter pylori* to make its way to the "safe" area-the protective mucous lining. Once there, the bacterium's spiral shape helps it burrow through the lining.

*Helicobacter pylori*-mediated peptic ulcers are treated with drugs that kill the bacteria, reduce stomach acid, and protect the stomach lining. Antibiotics are used to kill the bacteria. Three types of acid-suppressing drugs might be used: Antacids, H₂ blockers and proton pump inhibitors ((H+/K+ ATPase inhibitors). Antacids neutralize directly the gastric acid and H₂ blockers work by blocking histamine, which stimulates acid secretion. They help reduce ulcer pain after a few weeks. Proton pump inhibitors suppress acid production by halting the mechanism that pumps the acid into the stomach. H₂ blockers and proton pump inhibitors have been prescribed on their own for years as treatments for ulcers. But used alone, these drugs do not eradicate *Helicobacter pylori* and therefore do not cure *Helicobacter pylori*-related ulcers. Bismuth subsalicylate is used to protect the stomach lining from acid. It also kills *Helicobacter pylori.*

Treatment usually involves a combination of antibiotics, acid suppressors, and stomach protectors. Antibiotic regimens recommended for patients may differ across regions of the world because different areas have begun to show resistance to particular antibiotics. The use of only one medication to treat *Helicobacter pylori* is not recommended. At the moment, the most proven effective treatment is a 2-week course of treatment called triple therapy. It involves taking two antibiotics to kill the bacteria and either an acid suppressor or stomach-lining shield. Two-week triple therapy reduces ulcer symptoms, kills the bacteria, and prevents ulcer recurrence in more than 90 percent of patients.

WO9624375 claims an oral pharmaceutical dosage in a tablet form comprising an acid susceptible proton pump inhibitor together with at least one antibacterial compound in a fixed formulation. The fixed formulation is intended for oral use and in the form of a capsule or a multiple unit dosage form. The multiple unit dosage form is most preferred. The formulation is especially useful in the treatment of disorders associated with *Helicobacter* infections. This therapy is forced to use sufficiently small pellets to guarantee a uniform distribution of active ingredients inside the tablets. The bad taste of clarithromycin constitutes an important disadvantage in the use of the composition in granulated form.

WO9702020 and WO9702021 claim oral pharmaceutical compositions for treating a disorder caused by *Helicobacter* comprising a reversible proton pump inhibitor in combination with at least one antimicrobially-active ingredient, wherein at least part of the proton pump inhibitor is in slow-release form. The composition is in pellet or tablet form. This therapy is formulated in such a way that when the proton pump inhibitor is administered in a sustained release form, then an enhanced antibiotic activity over *Helicobacter* occurs. No unconventional pharmaceutical technology is disclosed in these documents.

WO03082241 claims a pharmaceutical composition comprising micronized clarithromycin, wherein the pharmaceutical composition exhibits improved dissolution characteristics relative to a pharmaceutical composition that includes unmicronized clarithromycin. The clarithromycin has a particle size less than 35 microns. A process for preparing an extended release tablet includes micronizing the clarithromycin; blending the micronized clarithromycin with one or more rate controlling polymers and pharmaceutically acceptable excipients; granulating the blend; and compressing to form a tablet.

WO03082248 claims a pharmaceutical composition including erythromycin A or a derivative thereof and alginic acid. The alginic acid provides taste masking of the erythromycin A or derivative. The erythromycin A derivative may be clarithromycin and the alginic acid may be one or both of alginic acid and its salt. The salt may be one or more of sodium alginate and calcium alginate. The pharmaceutical composition may further include one or more of a binder, a disintegrant, a flavoring agent, and a coating. The pharmaceutical composition also may include one or more active ingredients, including proton pump inhibitors and antibiotics.

WO2005082331 claims an extended release tablet for oral administration comprising clarithromycin and a pharmaceutically acceptable carrier, wherein the pharmaceutically acceptable carrier comprises a mixture of lactose and microcrystalline cellulose in a ratio ranging from 3:1 to 1:3. The pharmaceutical composition also may include one or more active ingredients, including proton pump inhibitors and antibiotics.

US6576625 claims liposomes, pharmacosomes, niosomes and biosomes suitable for targeted vesicular constructs for treatment of *Helicobacter pylori* infections and/or for cytoprotection. The composition contains lecthins, phospholipids, sterols, and one or more drugs. The drugs are selected from the group consisting of antibiotics, antiprotozoals, H₂ receptor antagonists, protectants, astringents, and antacids.

EP1452533 claims a compound which is a crystalline form Z of sodium rabeprazole as well a pharmaceutical composition comprising a prophylactically or therapeutically effective amount of said compound and one or more antimicrobial compounds. The composition is useful for preventing or treating a disease that is associated with excess gastric acid secretion, namely ulcer, gastro esophageal reflux disease, psoriasis or Zollinger-Ellison Syndrome.

In spite of the reported inventions, unfortunately, patients still find current triple therapy complicated because it involves taking as many as 20 pills a day, thus resulting in an increased risk of erroneous dosage. Also, the antibiotics used in triple therapy may cause mild side effects such as nausea, vomiting, diarrhea, dark stools, metallic taste in the mouth, dizziness, headache, and yeast infections in women. In this way, there is a need to get safer medicaments with increased patient compliance.

Among the compositions claimed in the cited patent references, no any one comprising a proton pump inhibitor, a clarithromycin compound and an amoxicillin compound has been sufficiently disclosed.

### Summary of the invention

The present invention is directed to pharmaceutical compositions comprising simultaneously a proton pump inhibitor (H+/K+ ATPase inhibitor), a clarithromycin compound and an amoxicillin compound in a novel pellet composition that substantially comprises a core containing the proton pump inhibitor covered by a cellulose derivative protective layer to avoid contact of omeprazole with the externally adjacent enteric layer that guarantees the stability of the active agent in gastric juice, a sealing layer and an external layer containing the clarithromycin compound and the amoxicillin compound, or alternatively two external layers containing each antibiotic compound independently preferably with the clarithromycin first. Resulting compositions show a surprising stability in spite of the recognized pharmaceutical incompatibility derived from the combination of active agents. Accordingly, the present invention provides pellet compositions filled into sachets for oral administration, containing the recommended doses of such components for the eradication of *Helicobacter pylori* bacterium. Subsequently, the present invention provides an improved method for the treatment of gastrointestinal infections caused or mediated by *Helicobacter pylori.* Thus, contrary to complexity of available combination therapies that tend to make the compliance rate low, the compositions of the present invention increase the patient compliance with the treatment regimen, decrease treatment failures and cut down related costs, besides reducing the possibility of confusion among tablets, capsules and different strengths.

### Detailed description of the invention

The present invention relates to novel pellet compositions for oral administration to be filled in sachets comprising:
a) from 5 to 40 mg of a proton pump inhibitor (H+/K+ ATPase inhibitor) per sachet;
b) from 50 to 1000 mg of a clarithromycin compound per sachet; and
c) from 100 to 2000 mg of an amoxicillin compound per sachet,
   characterized in that a pellet comprises:
   (i) a core comprising the proton pump inhibitor, made either through coating with a proton pump inhibitor of an inert core, or elaborating a spheronized mixed core containing the proton pump inhibitor;
   (ii) a protective layer;
   (iii) a polymethacrylate enteric layer;
   (iv) an optional protective sealing layer;
   (v)
      a) a pH stabilized coating layer comprising a blend of the clarithromycin and the amoxicillin compounds; or
      b) two pH stabilized coating layers comprising each the clarithromycin and the amoxicillin compounds independently, separated by another sealing layer if necessary; and
   (vi) a final external layer to protect and improve the taste of the formulation.

Preferably, the amount of proton pump inhibitor is 10 to 40 mg per sachet, more preferably 20 mg per sachet; the amount of the clarithromycin compound is 250 to 750 mg per sachet, more preferably 500 mg per sachet; and the amount of the amoxicillin compound is 250 to 2000 mg per sachet, more preferably 1000 mg per sachet.

Proton pump inhibitor (H+/K+ ATPase inhibitor) refers to the group consisting of an omeprazole compound, a lansoprazole compound, an esomeprazole compound, a pantoprazole compound, a rabeprazole compound or a tenatoprazole compound. In practice, an omeprazole compound is preferred. The omeprazole compound is selected from the group consisting of omeprazole, omeprazole magnesium salt and omeprazole sodium salt, and more preferably is omeprazole.

Clarithromycin compound refers to the group consisting of clarithromycin and pharmaceutically acceptable salts thereof. In the present invention clarithromycin is preferred.

Amoxicillin compound refers to the group consisting of amoxicillin, amoxicillin sodium salt and amoxicillin trihydrate. In the present invention amoxicillin trihydrate is preferred.

According to the present invention, the proton pump inhibitor coats an inert core (i.e., a core containing one or more excipients, without active agent) or alternatively is embed in a matrix core. In the present compositions, the coating layers are pH stabilized with a mixture of phosphoric acid sodium salts, citric acid, glycine and arginine. Further the coating layers may comprise tensioactive agents, antifoam agents, softening agents, filmogenic agents and layer forming agents.

Tensioactive agents suitable for use with the present invention include, but are not limited to, polysorbates and polyethoxylated castor oils, and mixtures thereof.

Antifoam agents suitable for use with the present invention include, but are not limited to, dimethicone and simethicone, and mixtures thereof.

Softening agents suitable for use with the present invention include, but are not limited to, acetyl tributyl citrate, dibutyl phthalate and polyethylene glycol, and mixtures thereof.

Filmogenic agents suitable for use with the present invention include, but are not limited to, polyvinylpyrrolidone, hydroxyethylcellulose and hypromellose, and mixtures thereof.

According to the present invention the pellet further comprises a final external layer, wherein the forming agents are selected from the group consisting of polymethacrylates, cellulose acetate phthalate and hypromellose phthalate, and mixtures thereof. Also the final external layer comprises diluent agents selected from the group consisting of mannitol, sucrose and xylitol, and mixtures thereof, and flavors.

Another aspect of the present invention is to provide a composition for use in the treatment of disorders associated with *Helicobacter* in a human or non-human mammal.

Another aspect of the present invention is to provide a composition for use in the treatment of a gastric disorder associated with *Helicobacter* in a human or non-human mammal.

Another aspect of the present invention is to provide a use of a composition for the manufacture of a medicament for the treatment of disorders associated with *Helicobacter* infections in a human or non-human mammal.

Another aspect of the present invention is to provide a use of a composition for the manufacture of a medicament for the treatment of a gastric disorder associated with *Helicobacter* infections in a human or non-human mammal.

As used herein, the term "mammal" shall refer to the Mammalia class or higher vertebrates. The term "mammal" includes, but is not limited to, a human.

The present invention also relates to a method of treatment or prevention of a human or non-human mammal suffering from a disorder associated with *Helicobacter* infections, which comprises administering to said human or non-human mammal in need thereof the composition of the present invention. More specifically, the disorder is a gastric disorder associated with *Helicobacter* infections.

### Manufacturing process

Amoxicillin and clarithromycin compounds are added to omeprazole containing cores previously coated with an enteric layer of polymethacrylate (Eudragit 30 D-55) according to the following operations:

a) Preparing the sealing layer. The purpose of the sealing layer is to prevent moisture and to separate the antibiotics from the enteric layer that protects omeprazole contained in the pellet, resulting in a thorough protection of the pellet and assuring the stability of the antibiotics and the medicament in general.

The preparation should be made in excess to adjust possible wastages during the application. To prepare a small-scale batch, we start from 50 g of hydroxypropylmethylcellulose (Pharmacoat 606) and deionized water (q.s. 500 mL) the process comprises the following operations given as a preparation example:
1 - Dissolve the hydroxypropylmethylcellulose in 500 mL of deionized water contained in a glass or stainless steel vessel by stirring with a stirrer until complete dissolution.
2 - Place the omeprazole pellets (300 g) on a fluid bed (Wurster) and proceed to coating according to the following specifications:

| | |
|---|---|
| Input air temperature | 60-65°C |
| Output air temperature | 45-50°C |
| Spray-gun pressure | 2.5-3 bar |
| Application pump speed | 1.2-1.5 rpm |
| Input air cursor position | 30-35 |

As a result of this process, a proper coating of the pellets is obtained.

The weight increase is 2 %. Therefore, 100 g of processed pellets contain 2 g of hydroxypropylmethylcellulose. The obtained pellets are stored in a container with a hermetic seal and protected against moisture and light until next use.

### b) Preparing the antibiotic layer

It can be prepared starting from the following components:

| | |
|---|---|
| Micronized clarithromycin | 135 g |
| Micronized amoxicillin trihydrate | 310 g |
| Povidone 90 F | 20 g |
| Polysorbate 80 | 1 g |
| Silicone oil | 1.60 g |
| Buffer solution (pH 6) | 1180 g |

The process comprises the following operations:
1 - Preparing the buffer solution (pH 6): Dissolve 143 g of sodium hydrogen phosphate in 2000 mL of deionized water. Dissolve 42 g of citric acid in 2000 mL of deionized water.
2 - Dissolve the povidone in 1180 g of buffer solution in a glass vessel provided with an anchor stirrer. Add the polysorbate and the silicone oil stirring until full homogeneity.
3 - Disperse the micronized clarithromycin and the amoxicillin stirring until full homogeneity. The pH of the obtained dispersion is 6.91.
4 - Pass the dispersion through a 0.100 mm mesh and collect the dispersion in another appropriate vessel, followed by further stirring during over the process.
5 - Place the omeprazole pellets (306 g) on a fluid bed (Wurster) and proceed to coating according to the following specifications:

| | |
|---|---|
| Input air temperature | 60-65°C |
| Output air temperature | 45-50°C |
| Spray-gun pressure | 2.5-3 bar |
| Application pump speed | 1.2-1.5 rpm |
| Input air cursor position | 30-35 |

As a result, a correct coating of pellets is obtained.

In this example given as a reference, the weight increase is about 132 %. Therefore in this case, 100 mg of processed pellets contain 35.93 mg of amoxicillin (trihydrate) and 15.65 mg of clarithromycin, although the amounts of antibiotics per pellets could be increased through successive separated steps. The obtained pellets are stored in a container with a hermetic seal and protected against moisture and light.

To verify the release of the antibiotics, a HPLC titration of the active ingredients and their degradation products as standard and a dissolution of the final formulation were performed using amoxicillin trihydrate as a tracer.

The results obtained are shown in Tables 1-2.

**Table 1**

| Amoxicillin and degradation product - | | | | | |
|---|---|---|---|---|---|
| Determinations | Amoxicillin % | Dp₁ | Dp₂ | Dp₃ | Dp₄ |
| 1 | 30.28 | 0.11 | 0.14 | 0.14 | 0.12 |
| 2 | 30.44 | 0.11 | 0.14 | 0.15 | 0.14 |
| 3 | 32.81 | 0.11 | 0.15 | 0.18 | 0.17 |
| Mean | 31.18 | 0.11 | 0.14 | 0.16 | 0.14 |
| SD | 1.414 | 0.004 | 0.008 | 0.017 | 0.025 |
| RSD | 4.536 | 3.580 | 5.918 | 11.137 | 17.410 |

| | | | | | |
|---|---|---|---|---|---|
| Dp₁ α-Hydroxy-D-phenylglycine Dp₂ Peniloic acids Dp₃ Peniciloics acids Dp₄ Amoxicillin diketopiperazine | | | | | |

Amoxicillin and its degradation product results are expressed in mg of active ingredient and mg of degradation products per 100 mg of pellets.

**Table 2**

| Dissolution test (% of dissolved amoxicillin) | | | | | | |
|---|---|---|---|---|---|---|
| Time | Vessel 1 | Vessel 2 | Vessel 3 | Vessel 4 | Vessel 5 | Vessel 6 |
| 15 min | 98.17 | 98.91 | 96.20 | 105.10 | 101.98 | 100.71 |
| 30 min | 98.93 | 97.71 | 96.34 | 96.86 | 96.89 | 98.24 |
| 45 min | 98.04 | 95.50 | 95.38 | 95.86 | 97.71 | 98.35 |

Moreover, the influence of the manufacturing process in the degradation of amoxicillin was studied by titrating the degradation products in the raw material and later on in a sample of the finished product. The obtained results are shown in Table 3.

**Table 3**

| Degradation products | % Dp raw material | % Dp finished product |
|---|---|---|
| Dp₁ | 0.09 | 0.11 |
| Dp₂ | 0.18 | 0.14 |
| Dp₃ | 0.07 | 0.16 |
| Dp₄ | 0.06 | 0.14 |

As depicted in Table 3, the amount of the degradation product 1 increases 0.02 % in the finished product when compared to the raw material. The increase in degradation products 3 is 0.09 % and for the degradation product 4 is 0.08 %. The increase of degradation products 3 and 4 suggests that the manufacturing process has some influence on the degradation of the product. For degradation products 2, its percentage is lower in the finished product than in the raw material because they degrade to other minor products.

The reported percentages for the degradation products are within the accepted 2 % for manufacturing processes. In conclusion, the manufacturing process is safe and does not affect significantly the expected composition of the obtained pellets.

### Example 1: Pellet composition comprising omeprazole 20 mg, an enteric layer of Eudragit 30 D-55 (polymethacrylate), a protective layer and a two antibiotic coating layer, to be filled in sachets for oral administration

| Sachet composition | | |
|---|---|---|
| Omeprazole enteric pellets | (Active ingredient) | 1354 mg |
| Hydroxypropylmethylcellulose | (Protective layer) | 26 mg |
| Sodium hydrogen phosphate | (pH adjusting agent) | 198 mg |
| Citric acid | (pH adjusting agent) | 33 mg |
| Povidone 90 F | (Filmogenic agent) | 74 mg |
| Dimethicone | (Antifoam agent) | 5.9 mg |
| Amoxicillin trihydrate | (Active ingredient) | 1148 mg |
| Clarithromycin | (Active ingredient) | 500 mg |
| Polysorbate 80 | (Tensioactive agent) | 3.6 mg |

The composition is manufactured according to the general method outlined in the Manufacturing process.

### Example 2: Pellet composition comprising omeprazole 20 mg, an enteric layer of polymethacrylate, a sealing layer, a two antibiotic coating layer, and a sugar and flavor final coating layer.

| Sachet composition | | |
|---|---|---|
| Omeprazole enteric pellets | (Active ingredient) | 1354 mg |
| Hydroxypropylmethylcellulose | (Protective layer) | 26 mg |
| Sodium hydrogen phosphate | (pH adjusting agent) | 198 mg |
| Citric acid | (pH adjusting agent) | 33 mg |
| Povidone 90 F | (Filmogenic agent) | 74 mg |
| Dimethicone | (Antifoam agent) | 5.9 mg |
| Amoxicillin trihydrate | (Active ingredient) | 1148 mg |
| Clarithromycin | (Active ingredient) | 500 mg |
| Polysorbate 80 | (Tensioactive agent) | 3.6 mg |
| Mannitol | (Diluent agent) | 605 mg |
| Strawberry flavor | (Flavoring agent) | 5 mg |

The composition is manufactured according to the general method outlined in the Manufacturing process. The mannitol and flavor final coating layer is added with a pan coater.

### Example 3: Pellet composition comprising some different pH adjusting agents

| Sachet composition | | |
|---|---|---|
| Omeprazole enteric pellets | 1354 mg | |
| Hydroxypropylmethylcellulose | 20 mg | Sealing layer |
| Amoxicillin trihydrate | 1148 mg | Stabilized antibiotics containing coating layer |
| Clarithromycin | 500 mg | |
| Glycine | 10 mg | |
| Arginine | 5 mg | |
| Povidone 90 F | 70 mg | |
| Polysorbate 80 | 5 mg | |
| Dimethicone | 5 mg | |
| Hydroxypropylmethylcellulose | 20 mg | Protective layer |

The composition is manufactured according to the general method outlined in the Manufacturing process.

### Example 4: Pellet composition comprising some different pH adjusting agents and a mannitol filling layer

| Sachet composition | | |
|---|---|---|
| Omeprazole enteric pellets | 1354 mg | |
| Hydroxypropylmethylcellulose | 20 mg | Sealing layer |
| Amoxicillin trihydrate | 1148 mg | Stabilized antibiotics containing coating layer |
| Clarithromycin | 500 mg | |
| Glycine | 10 mg | |
| Arginine | 5 mg | |
| Povidone 90 F | 70 mg | |
| Polysorbate 80 | 5 mg | |
| Dimethicone | 5 mg | |
| Hydroxypropylmethylcellulose | 20 mg | Protective layer |
| Mannitol | 605 mg | Final coating layer Final coating layer |
| Strawberry flavor | 5 mg | |

The composition is manufactured according to the general method outlined in the Manufacturing process. The mannitol and flavor final coating layer is added with a pan coater.

### Example 5: Pellet composition comprising some different tensioactive agents in order to improve the antibiotics release

| Sachet composition | | |
|---|---|---|
| Omeprazole enteric pellets | 1354 mg | |
| Hydroxypropylmethylcellulose | 20 mg | Sealing layer |
| Amoxicillin trihydrate | 1148 mg | Stabilized antibiotics containing coating layer |
| Clarithromycin | 500 mg | |
| Glycine | 10 mg | |
| Arginine | 5 mg | |
| Povidone 90 F | 70 mg | |
| Polysorbate 80 | 5 mg | |
| Cremophor RH40 | 20 mg | |
| Dimethicone | 5 mg | |
| Hydroxypropylmethylcellulose | 20 mg | Protective layer |

The composition is manufactured according to the general method outlined in the Manufacturing process.

### Example 6: Pellet composition comprising some different tensioactive agents and a mannitol final coating layer

| Sachet composition | | |
|---|---|---|
| Omeprazole enteric pellets | 1354 mg | |
| Hydroxypropylmethylcellulose | 20 mg | Sealing layer |
| Amoxicillin trihydrate | 1148 mg | Stabilized antibiotics containing coating layer |
| Clarithromycin | 500 mg | |
| Sodium hydrogen phosphate | 200 mg | |
| Citric acid | 35 mg | |
| Glycine | 20 mg | Stabilized antibiotics containing coating layer |
| Povidone 90 F | 70 mg | |
| Polysorbate 80 | 5 mg | |
| Cremophor RH40 | 20 mg | |
| Dimethicone | 5 mg | |
| Hydroxypropylmethylcellulose | 20 mg | Protective layer |
| Mannitol | 605 mg | Final coating layer |
| Orange flavor | 10 mg | |

The composition is manufactured according to the general method outlined in the Manufacturing process. The mannitol and flavor final coating layer is added with a pan coater.

### Example 7: Pellet composition comprising some different softening agents and filmogenic agents

| Sachet composition | | |
|---|---|---|
| Omeprazole enteric pellets | 1352 mg | |
| Hydroxypropylmethylcellulose | 20 mg | Sealing layer |
| Dibutyl phthalate | 5 mg | |
| Amoxicillin trihydrate | 1148 mg | Stabilized antibiotics containing coating layer |
| Clarithromycin | 500 mg | |
| Sodium hydrogen phosphate | 200 mg | |
| Citric acid | 35 mg | |
| Glycine | 20 mg | |
| Polyethylene glycol 4000 | 10 mg | |
| Polysorbate 20 | 5 mg | |
| Dimethicone | 5 mg | |
| Polyvinylpyrrolidone K-30 | 20 mg | Final coating layer |
| Dibutyl phthalate | 2 mg | |

The composition is manufactured according to the general method outlined in the Manufacturing process.

### Example 8: Pellet composition comprising some different softening agents and filmogenic agents and a mannitol final coating layer

| Sachet composition | | |
|---|---|---|
| Omeprazole enteric pellets | 1352 mg | |
| Hydroxypropylmethylcellulose | 20 mg | Sealing layer |
| Dibutyl phthalate | 5 mg | |
| Amoxicillin trihydrate | 1148 mg | Stabilized antibiotics containing coating layer |
| Clarithromycin | 500 mg | |
| Sodium hydrogen phosphate | 200 mg | |
| Citric acid | 35 mg | |
| Glycine | 20 mg | |
| Polyethylene glycol 4000 | 70 mg | |
| Polysorbate 20 | 5 mg | |
| Dimethicone | 5 mg | |
| Polyvinylpyrrolidone K-30 | 20 mg | Sealing layer |
| Dibutyl phthalate | 2 mg | |
| Mannitol | 605 mg | Final coating layer |
| Orange flavor | 10 mg | |

The composition is manufactured according to the general method outlined in the Manufacturing process. The mannitol and flavor final coating layer is added with a pan coater.

### Example 9: Pellet composition comprising a sucrose final coating layer

| Sachet composition | | |
|---|---|---|
| Omeprazole enteric pellets | 1352 mg | |
| Hydroxypropylmethylcellulose | 20 mg | Sealing layer |
| Amoxicillin trihydrate | 1148 mg | Stabilized antibiotics containing coating layer |
| Clarithromycin | 500 mg | |
| Arginine | 10 mg | |
| Glycine | 10 mg | |
| Polyethylene glycol 4000 | 10 mg | |
| Cremophor RH40 | 20 mg | |
| Dimethicone | 5 mg | |
| Hydroxypropylmethylcellulose | 20 mg | Sealing layer |
| Dibutyl phthalate | 2 mg | |
| Sucrose | 500 mg | Final coating layer |
| Dibutyl phthalate | 3 mg | |

The composition is manufactured according to the general method outlined in the Manufacturing process. The sucrose final coating layer is added with a pan coater.

### Example 10: Pellet composition comprising a xylitol final coating layer

| Sachet composition | | |
|---|---|---|
| Omeprazole enteric pellets | 1354 mg | |
| Hydroxypropylmethylcellulose | 20 mg | Sealing layer |
| Amoxicillin trihydrate | 1148 mg | Stabilized antibiotics containing coating layer |
| Clarithromycin | 500 mg | |
| Arginine | 10 mg | |
| Glycine | 10 mg | |
| Polyethylene glycol 4000 | 10 mg | |
| Polysorbate 80 | 15 mg | |
| Dimethicone | 5 mg | |
| Hydroxypropylmethylcellulose | 20 mg | Sealing layer |
| Xylitol | 600 mg | Final coating layer |
| Polyvinylpyrrolidone K-90 | 5 mg | |

The composition is manufactured according to the general method outlined in the Manufacturing process. The xylitol final coating layer is added with a pan coater.

## Claims

1. A pellet composition for oral administration to be filled in sachets comprising:
a) from 5 to 40 mg of a proton pump inhibitor (H+/K+ ATPase inhibitor) per sachet;
b) from 50 to 1000 mg of a clarithromycin compound per sachet; and
c) from 100 to 2000 mg of an amoxicillin compound per sachet,
**characterized in that** a pellet comprises:
(i) a core comprising the proton pump inhibitor, made either through coating with a proton pump inhibitor of an inert core, or elaborating a spheronized mixed core containing the proton pump inhibitor;
(ii) a protective layer;
(iii) a polymethacrylate enteric layer;
(iv) an optional protective sealing layer;
(v)
a) a pH stabilized coating layer comprising a blend of the clarithromycin and the amoxicillin compounds; or
b) two pH stabilized coating layers comprising each the clarithromycin and the amoxicillin compounds independently, separated by another sealing layer if necessary; and
(vi) a final external layer to protect and improve the taste of the formulation.

2. The composition according to claim 1 wherein:
a) the amount of proton pump inhibitor is 10 to 40 mg per sachet;
b) the amount of the clarithromycin compound is 250 to 750 mg per sachet; and
c) the amount of the amoxicillin compound is 250 to 2000 mg per sachet.

3. The composition according to claim 2 wherein:
a) the amount of proton pump inhibitor is 20 mg per sachet;
b) the amount of the clarithromycin compound is 500 mg per sachet; and
c) the amount of the amoxicillin compound is 1000 mg per sachet.

4. The composition according to claims 1-3 wherein the proton pump inhibitor is selected from the group consisting of an omeprazole compound, a lansoprazole compound, an esomeprazole compound, a pantoprazole compound, a rabeprazole compound and a tenatoprazole compound.

5. The composition according to claim 4 wherein the proton pump inhibitor is an omeprazole compound.

6. The composition according to claim 5 wherein the omeprazole compound is selected from the group consisting of omeprazole, omeprazole magnesium salt and omeprazole sodium salt.

7. The composition according to claim 6 wherein the proton pump inhibitor compound is omeprazole.

8. The composition according to claims 4-7 wherein the clarithromycin compound is clarithromycin.

9. The composition according to claims 4-8 wherein the amoxicillin compound is selected from the group consisting of amoxicillin, amoxicillin sodium salt and amoxicillin trihydrate.

10. The composition according to claim 9 wherein the amoxicillin compound is amoxicillin trihydrate.

11. The composition according to claim 1 wherein the coating layers are pH stabilized with a mixture of phosphoric acid sodium salts, citric acid, glycine and arginine.

12. The composition according to claim 1 wherein the coating layers further comprise tensioactive agents.

13. The composition according to claim 1 wherein the coating layers further comprise antifoam agents.

14. The composition according to claim 1 wherein the coating layers further comprise softening agents and filmogenic agents.

15. The composition according to claim 1 wherein the enteric layer further comprises enteric layer forming agents.

16. The composition according to claim 12 wherein the tensioactive agents are selected from the group consisting of polysorbates and polyethoxylated castor oils, and mixtures thereof.

17. The composition according to claim 13 wherein the antifoam agents are selected from the group consisting of dimethicone and simethicone, and mixtures thereof.

18. The composition according to claim 14 wherein the softening agents are selected from the group consisting of acetyl tributyl citrate, dibutyl phthalate and polyethylene glycol, and mixtures thereof, and the filmogenic agents are selected from the group consisting of polyvinylpyrrolidone, hydroxyethylcellulose and hypromellose, and mixtures thereof.

19. The composition according to claim 15 wherein the enteric layer forming agents are selected from the group consisting of polymethacrylates, cellulose acetate phthalate and hypromellose phthalate, and mixtures thereof.

20. The composition according to claim 1 wherein the final external layer comprises diluent agents selected from the group consisting of mannitol, sucrose and xylitol, and mixtures thereof, and flavors.

21. A composition as defined in claims 1-20 for use in the treatment of disorders associated with *Helicobacter* in a human or non-human mammal.

22. The composition according to claim 21 wherein the disorder is a gastric disorder associated with *Helicobacter* in a human or non-human mammal.

23. Use of a composition as defined in claims 1-20 for the manufacture of a medicament for the treatment of disorders associated with *Helicobacter* infections in a human or non-human mammal.

24. The use of a composition according to claim 23 wherein the disorder is a gastric disorder associated with *Helicobacter.*
